(19) Europäisches Patentamt
European Patent Office
Office européen des brevets

(11) Publication number: 0 116 372 B1

(12) EUROPEAN PATENT SPECIFICATION

(45) Date of publication of patent specification: 21.01.87

(21) Application number: 84101519.1

(22) Date of filing: 14.02.84

(51) Int. Cl.[4]: C 07 D 498/04, A 61 K 31/535 // C07D313/08 ,(C07D498/04, 313:00, 265:00)

(54) 2H-(1) benzoxepino (5,4-b)-1,4 oxazine derivatives.

(30) Priority: 15.02.83 US 466442
21.12.83 US 562757

(43) Date of publication of application:
22.08.84 Bulletin 84/34

(45) Publication of the grant of the patent:
21.01.87 Bulletin 87/04

(84) Designated Contracting States:
AT BE CH DE FR GB IT LI LU NL SE

(56) References cited:
GB-A-1 211 258

(73) Proprietor: MERRELL DOW PHARMACEUTICALS INC.
2110 E. Galbraith Road
Cincinnati Ohio 45215 (US)

(72) Inventor: Freedman, Jules
10553 Adventure Lane
Cincinnati, OH 45242 (US)

(74) Representative: Vossius & Partner
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 116 372 B1

Courier Press, Leamington Spa, England.

**Description**

Benzoxepine derivatives containing an amino group in 4-position of the oxepine ring are known from GB—A—1,211,258. These compounds are said to exhibit antidepressive and anticonvulsive actions.

This invention relates to 2*H*-[1]benzoxepino[5,4-*b*]-1,4-oxazine derivatives which may be substituted at the phenyl ring or at the amine nitrogen atom. More particularly, this invention relates to the *cis* and *trans* (4a,11b)-3,4,4a,5,6,11b-hexahydro-2*H*-[1]benzoxepino[5,4-*b*]-1,4-oxazine derivatives having the following general formula I

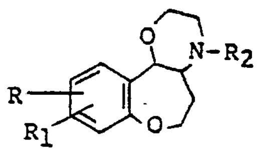

(I)

wherein R and $R_1$ are each hydrogen, fluoro, chloro and $C_1$—$C_4$-alkyl; $R_2$ is hydrogen, $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl or

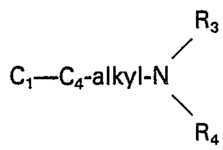

wherein $R_3$ and $R_4$ are each hydrogen or $C_1$—$C_4$-alkyl; and the pharmaceutical acceptable salts thereof. These derivatives possess useful analgesic and muscle relaxant activity. This invention further discloses a process whereby these derivatives can be conveniently prepared and in good yield.

Preferred are the compounds of the general formula I wherein $R_2$ is hydrogen or $C_1$—$C_4$-alkyl and the compounds wherein R and $R_1$ are hydrogen.

As seen in general formula (I) above, the benzenoid moiety ring of the benzoxepino moiety can be substituted or unsubstituted as indicated by the symbols R and $R_1$. When substituted, the various substituents can be located at any one of the four positions on the benzenoid moiety, i.e., positions 8, 9, 10 or 11. The various substituents that are encompassed as being within the scope of the invention include the fluoro, chloro and $C_1$—$C_4$-alkyl groups either alone or in combination.

In addition, the ring nitrogen atom may be substituted or remain unsubstituted as indicated by the symbol $R_2$. The various substituents for the symbol $R_2$ that are included within the scope of this invention are the $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl, the

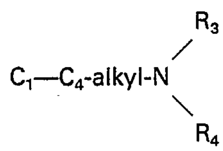

groups and those equivalents thereof that are known to the art. The symbols $R_3$ and $R_4$ represent either hydrogen or a $C_1$—$C_4$-alkyl radical and can be either N-$C_1$—$C_4$-alkyl or N,N-di-$C_1$—$C_4$-alkyl substituted. The N,N-di-$C_1$—$C_4$-alkyl group may be substituted with either the same $C_1$—$C_4$-alkyl group, or it may be substituted with a mixed $C_1$—$C_4$-alkyl group.

Examples for $C_1$—$C_4$-alkyl radicals are the methyl, ethyl, propyl, isopropyl, butyl, isobutyl or *t*-butyl radicals. The expression phenyl-$C_1$—$C_4$-alkyl relates to the 1,4-oxazine ring nitrogen being substituted by a $C_1$—$C_4$-alkyl group which terminates in a phenyl group, as for example, benzyl, phenethyl, phenpropyl, phenbutyl, etc.

The term $C_1$—$C_4$-alkoxy-$C_1$—$C_4$-alkyl represents those groups wherein the 1,4-oxazine ring nitrogen is substituted by a $C_1$—$C_4$-alkyl group which terminates in a $C_1$—$C_4$-alkyl ether, as for example, the 2-methoxyethyl, 3-ethoxypropyl and 4-propoxybutyl groups.

The term

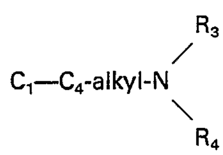

as used herein relates to those $C_1$—$C_4$-alkyl groups attached to the 1,4-oxazine ring nitrogen which terminate as either a primary, secondary or tertiary amine. Illustrative of such groups are the ethanamine, 2-propanamine, N-ethyl-ethanamine, N,N-dimethyl-ethanamine or N-methyl-N-propyl-3-propanamine groups.

The expression pharmaceutically acceptable salts refer to those non-toxic organic or inorganic acid addition salts which are equivalent to the above amines for the purposes of this invention. Illustrative inorganic acids which form suitable salts are hydrochloric, hydrobromic, sulfuric and phosphoric acid as well as acid metal salts such as sodium monohydrogen orthophosphate and potassium hydrogen sulfate. Illustrative organic acids which form suitable salts include the mono-, di- and tri-carboxylic acids, for example, acetic, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Either the mono- or the di-acid salts can be formed, and such salts can exist in either a hydrated or a substantially anhydrous form.

Illustrative compounds encompassed by the present invention include:
(4a,11b)-10-ethyl-3,4,4a,5,6,11b-hexahydro-4-propyl-2*H*-[1]benzoxepino[5,4-*b*]-1,4-oxazine;
(4a,11b)-8-butyl-9-fluoro-4-(3-ethoxypropyl)-3,4,4a,5,6,11b-hexahydro-2*H*-[1]benzoxepino[5,4-*b*]-1,4-oxazine;
(4a,11b)-3,4,4a,5,6,11b-hexahydro-9-(2-methylpropyl)-4-phenethyl-10-propyl-2*H*-[1] benzoxepino[5,4-*b*]-1,4-oxazine;
(4a,11b)-4-(2-furanylmethyl)-3,4,4a,5,6,11b-hexahydro-9-propyl-2*H*-[1]benzoxepino[5,4-*b*]-1,4-oxazine;
(4a,11b)-4-dimethylaminoethyl-3,4,4a,5,6,11b-hexahydro-8,9-diethyl-2*H*-[1]benzoxepino[5,4-*b*]-1,4-oxazine;
(4a,11b)-4-cyclopropylmethyl-3,4,4a,5,6-11b-hexahydro- 2*H*-[1]benzoxepino[5,4-*b*]-1,4-oxazine;
(4a,11b)-3,4,4a,5,6,11b-hexahydro-8,9-dichloro-4-(2-methylpropyl)-2*H*-[1]benzoxepino[5,4-*b*]-1,4-oxazine;
said compounds being either in a *cis* or *trans* configuration.

The novel (4a,11b)-3,4,4a,5,6,11b-hexahydro-2*H*-[1]benzoxepino[5,4-*b*]-1,4-oxazines of formula (I) can be readily prepared as illustrated in the following reaction scheme, wherein the symbols R, $R_1$ and $R_2$ are as defined above, except that $R_2$ cannot be hydrogen.

Reaction Scheme A:

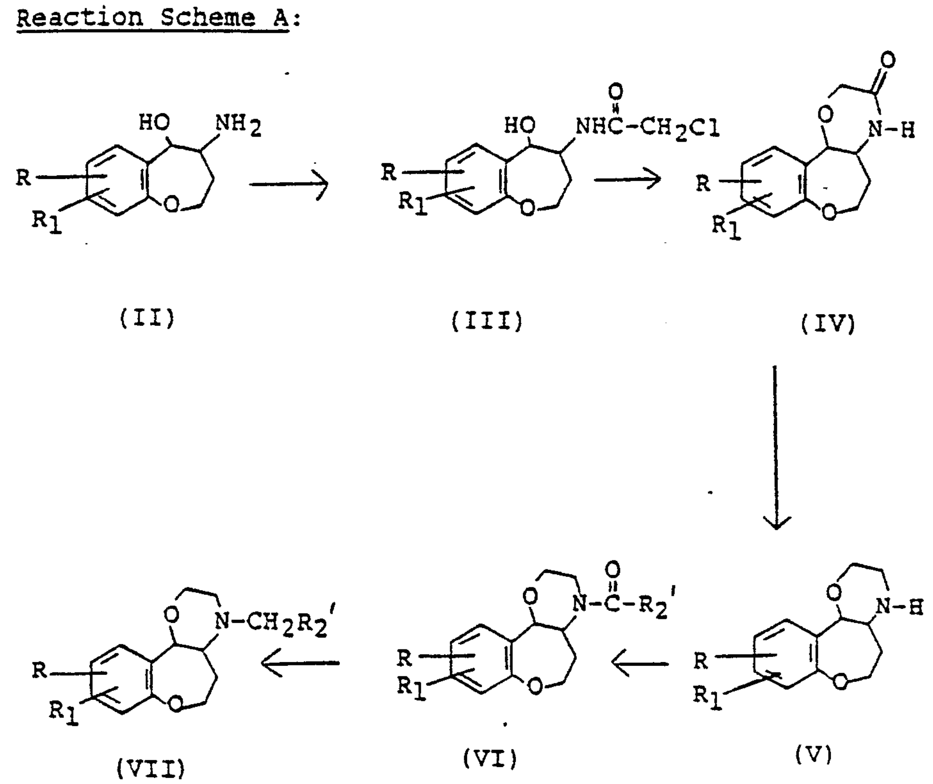

(II) (III) (IV)

(VII) (VI) (V)

In the formulae VI and VII $R_2'$ is defined as $R_2$ above, however containing one methylene group less than $R_2$.

Thus, a substituted 4-amino-2,3,4,5-tetrahydro-1-benzoxepin-5-ol (II) can be acylated with chloroacetyl chloride in the presence of a trialkylamine, such as triethylamine to yield the corresponding substituted 2'-chloro-*N*-(2,3,4,5-tetrahydro-5-hydroxy-1-benzoxepin-4-yl)acetamide (III). The acylation can be conducted

in an inert, halogenated solvent such as chloroform or methylene chloride at a temperature ranging from about 10° to about 30°C.

The chloroacetamido alcohol (III) can be cyclized with strong alkali in an aqueous alcohol solution. More particularly, cyclization can occur using a concentrated solution (50%) of sodium hydroxide in isopropanol or aqueous-isopropanol at room temperature for a period of from 8 to 24 hours.

The substituted (4a,11b)-4a,5,6,11b-tetrahydro-2*H*-[1]benzoxepino[5,4-*b*]-1,4-oxazine-3-(4H)one (IV) so obtained is reduced using a hydride reagent or diborane in an inert organic solvent. More particularly, lithium aluminum hydride can be favorably employed in a refluxing solvent, such as tetrahydrofuran or diethyl ether for a period of from about 3 to 12 hours. Where the symbol $R_2$ represents hydrogen in formula (I) above, the substituted (4a,11b)-3,4,4a,5,6,11b-hexahydro-2*H*-[1]benzoxepino[5,4-*b*]-1,4-oxazines (V) are obtained.

Alternatively, where the symbol $R_2$ is other than hydrogen or methyl in formula (I) above, the resulting substituted (4a,11b)-3,4,4a,5,6,11b-hexahydro-2*H*-[1]benzoxepino[5,4-*b*]-1,4-oxazines (V) are conveniently acylated with an acid chloride or an acid anhydride in the presence of a trialkylamine. Acylation can again be conducted in the presence of an inert halogenated solvent such as chloroform or methylene chloride to provide the corresponding substituted-*N*-acyl-(4a,11b)-3,4,4a,5,6,11b-hexahydro-2*H*-[1]benzoxepino[5,4-*b*]-1,4-oxazines (VI).

Reduction of these *N*-acyl-2*H*-[1]benzoxepino[5,4-*b*]-1,4-oxazines (VI) using a hydride or diborane reagent provides the desired (4a,11b)-3,4,4a,5,6,11b-hexahydro-2*H*-[1]benzoxepino[5,4-*b*]-1,4-oxazines (VII), wherein the symbol $R_2$ is other than hydrogen. More particularly, lithium aluminum hydride can be favorably employed in an inert, refluxing solvent, such as tetrahydrofuran or diethyl either for a period of from about 3 to 12 hours.

Alternatively, where the symbol $R_2$ in formula I represents a methyl group, it may be preferable to reduce the *N*-carboxylic acid ester in lieu of the *N*-acyl derivative in the last step of the above-indicated reaction sequence. Thus, the substituted (4a,11b)-3,4,4a,5,6,11b-hexahydro-2*H*-[1]benzoxepino[5,4-*b*]-1,4-oxazines (V) can be reacted with an alkyl chloroformate in the presence of a trialkylamine and a solvent such as chloroform or methylene chloride to provide the corresponding substituted (4a,11b)-3,4,4a,5,6,11b-hexahydro-4-carboalkoxy-2*H*-[1]benzoxepino[5,4-*b*]-1,4-oxazine derivatives (VIII) having the general formula

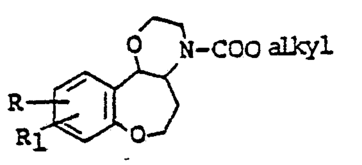

(VIII)

Reduction of this 4-carboalkoxy derivative with a hydride or diborane reagent, using essentially the same procedure as for the reduction of the *N*-acyl-(4a,11b)-3,4,4a,5,6,11b-hexahydro-2*H*-[1]benzoxepino[5,4-*b*]-1,4-oxazine (VI) provides the desired 4-methyl derivative.

It is to be noted that the intermediate 4-amino-2,3,4,5-tetrahydrobenzoxepin-5-ol compounds (II) may exist in either their *cis*- or *trans*- geometric isomeric forms, each of which are enantiomeric mixtures which may be separated into individual enantiomers by methods known in the art such as by the formation of diastereomeric salts. Alternatively, each enantiomer can be synthesized from an optically pure starting material. All such isomers are embraced herein although throughout this specification it is preferred, from an end-use application of the compounds of this invention (I), to utilize the *trans* isomers.

The substituted 4-amino-2,3,4,5-tetrahydro-1-benzoxepin-5-ol compounds (II) useful as starting materials are either known compounds or compounds that can be readily prepared from known phenols or substituted phenols in accordance with the following reaction scheme:

Reaction Scheme B:

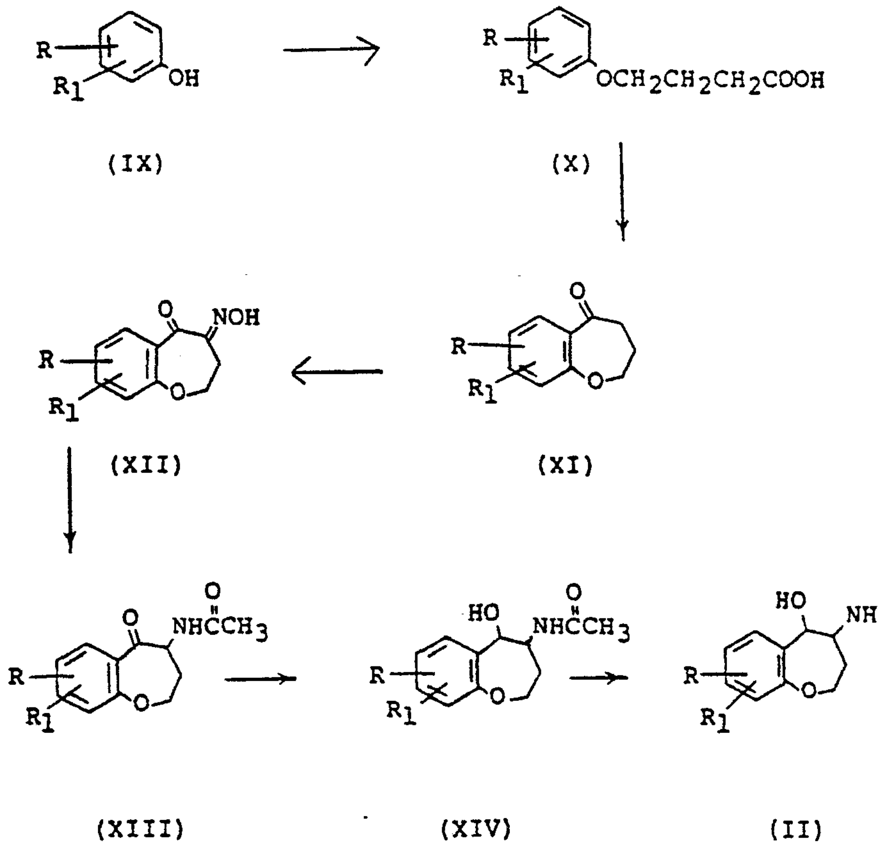

(IX) (X) (XI) (XII) (XIII) (XIV) (II)

Thus, phenol or a substituted phenol (IX) can be converted to the corresponding phenyloxybutyric acids (X) by heating the corresponding sodium phenolate with a slight excess of butyrolactone at a temperature of about 150—55°C. These phenyloxybutyric acids can be cyclized by heating with polyphosphoric acid at temperatures from about 55 to 100°C to yield the corresponding 2,3,4,5-tetrahydro-1-benzoxepin-5-ones (XI). The 2,3,4,5-tetrahydro-1-benzoxepin-5-ones so obtained (XI) can be dissolved in a solution of sodium ethoxide in ethanol and treated with isoamyl nitrite at ice-bath temperatures to form the 2,3,4,5-tetrahydro-1-benzoxepin-4,5-dione-4 oximes (XII).

The oximino ketones (XII) obtained in this manner can be reduced with zinc dust in an acetic acid/acetic anhydride mixture at temperatures of about 50—65°C to form the corresponding 2,3,4,5-tetrahydro-4-acetamido-1-benzoxepin-5-ones (XIII). Further reduction of these compounds by means of sodium borohydride in ethanol at temperatures ranging from about 10 to 30°C results in the formation of a mixture of the corresponding *cis*- and *trans*-2,3,4,5-tetrahydro-4-acetamido-1-benzoxepin-5-ols (XIV). Recrystallization of this mixture from a solvent such as ethyl acetate or acetonitrile results in the isolation of the desired *trans*-isomers. Hydrolysis of these isomers (XIV) by means of refluxing solution of aqueous sodium hydroxide in ethanol provides the desired *cis* or *trans* (or mixtures thereof) isomers of the 4-amino-2,3,4,5-tetrahydro-1-benzoxepin-5-ols (II), useful as starting materials for the preparation of the compounds of the present invention.

The compounds of this inventin diminish skeletal muscle tone as demonstrated by their antagonism to the so-called morphine-induced Straub tail, K. O. Ellis and J. F. Carpenter, Neuropharmacology *13*, 211—14 (1974). Accordingly, the compounds of this invention possess useful muscle relaxant activity and can be used in treating warm-blooded animals in the same manner as known muscle relaxants such as diazepam or mephenesin with due regard being given to the appropriate adjustment of dosages in accordance with the varying activities of these compounds.

In addition, the compounds of this invention have also been found to be useful for the amelioration of pain in warm-blooded animals as measured by the "writhing syndrome" test for analgesic activity as described by B. A. Whittle, Brit. J. Pharmacol. *22*, 2246 (1964).

In their end-use applications, either as muscle relaxants or as analgesic agents, the compounds most preferably are utilized in their *trans* isomeric form, although it is not essential that the administration of the preferred form be such that it be free of any of the *cis* form.

The term warm-blooded animals as used herein encompasses such species as mice, rats, guinea pigs,

rabbits, ferrets, dogs, cows, horses and primates including man.

The administration of these compounds can be carried out either via a parenteral route, such as by intravenous, intramuscular or intraperitoneal injection. Alternatively, the compounds disclosed herein can be introduced into the gastrointestinal tract vie oral administration, there to be absorbed into the blood stream. Alternatively, these compounds can be introduced to mammals in need thereof via intratracheal administration, such as by inhalation of a solution of the drug in the form of a spray.

An effective muscle relaxant or analgesic amount is that amount of drug substance which is sufficient to diminish skeletal muscle tone or effect an analgesic response in patients in need thereof. The particular amount of compound employed will vary widely depending upon various factors such as size, type, sex and age of the mammal to be treated, in addition to the mode and frequency of administration, the compound utilized or the particular pharmaceutically acceptable salt employed as well as the degree of hypertension to be treated. In particular instances, the dosage to be administered can be determined via conventional range finding techniques, as for example, by monitoring the reduction in blood pressure at various dose levels.

The compounds herein described can be administered at dosages ranging from about 3 mg to about 3000 mg of a 2*H*-[1]benzoxepino[5,4-*b*]-1,4-oxazine derivative, which can be administered from one to four times daily. More particularly, oral dosages of from about 1 to about 20 milligrams per kilogram of animal body weight can be employed. Slightly lower parenteral dosages of from about 0.1 mg to about 10 milligrams per kilogram of animal body weight can be favorably employed.

It is generally desirable to administer the compounds of this invention in dosage unit form. A unit dosage may contain from about 1 to 500 mg of active ingredients, preferably from 5 to 150 mg of active ingredient, and can be taken one or more times per day. Dosage units suitable for oral administration include tablets, capsules, lozenges, elixirs, syrups and the like.

The active compound can be formulated via conventional procedures or as a timed release capsule or tablet formulations using techniques well known to those skilled in the art. Where rapid action is desired, the active ingredients may be formulated as injectable compositions, sprays or aerosols for inhalation therapy.

In the practice of this invention, the active ingredient is preferably formulated as compositions comprising from about 5 percent to about 90 percent by weight of the particular 2*H*-[1]benzoxepino[5,4-*b*]-1,4-oxazine, or a pharmaceutically acceptable salt thereof sought to be administered, in combination with a pharmaceutical carrier.

The term pharmaceutical carrier refers to those pharmaceutical excipients known to the art which are non-toxic and which are useful in the formulation of pharmaceutical compositions. Such compositions can be prepared via techniques known to those skilled in the art for the preparation of tablets, capsules, lozenges, troches, suppositories, elixirs, syrups, emulsions, dispersions, wettable and effervescent powders, sterile injectable compositions and solutions for sprays, and can contain suitable excipients known to be useful in the preparations of the particular type of composition desired. Suitable pharmaceutical carriers and formulation techniques are described in standard texts such as Remington's Pharmaceutical Sciences, 16th Edition (1980), Mack Publishing Company, Easton, Pennsylvania.

The following examples are provided to further illustrate the present invention, by should not be construed as limiting the invention in any way.

Example 1

Trans-(4a,11b)-3,4,4a,5,6,11b-Hexahydro-2H-[1]benzoxepino[5,4-b]-1,4-oxazine Hydrochloride

To a solution of 8.07 g of *trans*-4-amino-2,3,4,5-tetrahydro-1-benzoxepin-5-ol and 9 ml of triethylamine in 400 ml of chloroform is slowly added a solution of 5.58 g of chloroacetyl chloride in 50 ml of chloroform. The mixture is permitted to stand overnight at room temperature, washed with a dilute hydrochloric acid solution and filtered through a bed of magnesium sulfate. Evaporation of the filtrate and a recrystallization of the residue from toluene yielded 8.43 g of *trans*-2'-chloro-*N*-(2,3,4,5-tetrahydro-5-hydroxy-1-benzoxepin-4-yl)acetamide having a m.pt. of 141—4°C.

A solution of 8.3 g of the above amide dissolved in 460 ml of isopropanol is treated with 7.2 g of a 50% aqueous sodium hydroxide solution and the mixture stirred overnight at room temperature. The solvent is concentrated and diluted with water. The insoluble material which forms is removed by filtration and recrystallized from acetonitrile to yield 4.31 g of *trans*-(4a,11b)-4a,5,6,11b-tetrahydro-2*H*-[1]benzoxepino[5,4-*b*]-1,4-oxazine-3(4H)one having a m.pt. of 246—8°C.

To a suspension of 1.5 g of lithium aluminum hydride in 100 ml of tetrahydrofuran is added, portionwise, 4.18 g of (4a,11b)-4a,5,6,11b-tetrahydro-2H-[1]benzoxepino[5,4-*b*]-1,4-oxazine-3(4H)one. The mixture is refluxed for five hours, cooled in ice and excess hydride decomposed by the addition of 6 ml of a 10% sodium hydroxide solution. The resulting mixture is stirred overnight, the solids removed by filtration and the solvent removed by evaporation. The residue is dissolved in ether and treated with ethereal hydrogen chloride. The title compound which precipitates is recrystallized from a methanol/acetonitrile solution to yield 2.69 g of material having a m.pt. of 259—60°C.

Following essentially the same procedure but substituting the following *trans*-4-amino-2,3,4,5-tetrahydro-1-benzoxepin-5-ols results in the following products shown below.

| Reactant | Product | m. pt. |
| --- | --- | --- |
| *trans*-4-amino-7-chloro-tetrahydro-1-benzoxepin-5-ol | (4a, 11b)-10-chloro-3,4,4a,5,6-11b-hexahydro-2H-[1]-benzoxepino[5,4-*b*]-1,4-oxazine hydrochloride | 283—4°C (dec.) |
| *trans*-4-amino-7-fluoro-2,3,4,5-tetrahydro-1-benzoxepin-5-ol | (4a,11b)-10-fluoro-3,4,4a,5,6,11b-hexahydro-2*H*-[1]benzoxepino-[5,4-*b*]-1,4-oxazine hydrochloride | 245—50°C (dec.) |
| *trans*-4-amino-8-chloro-2,3,4,5-tetrahydro-1-benzoxepin-5-ol | (4a,11b)-9-chloro-3,4,4a,5,6,11b-hexahydro-2*H*-[1]benzoxepino[5,4-*b*]-1,4-oxazine hydrochloride | 296—8°C (dec.) |
| *trans*-4-amino-7-(*t*-butyl)-2,3,4,5-tetrahydro-1-benzoxepin-5-ol | (4a,11b)-10-(*t*-butyl)-3,4,4a,5,6,11b-hexahydro-2*H*-[1]benzoxepino[5,4-*b*]-1,4-oxazine hydrochloride | 293—7°C (dec.) |
| *trans*-4-amino-7-methyl-2,3,4,5-tetrahydro-1-benzoxepin-5-ol | (4a,11b)-10-methyl-3,4,4a,5,6,11b-hexahydro-2*H*-[1]benzoxepino[5,4-*b*]-1,4-oxazine hydrochloride | 302—3°C (dec.) |

## Example 2

Trans-(4a,11b)-3,4,4a,5,6,11b-Hexahydro-4-methyl-2H-[1]benzoxepino[5,4-b]-1,4-oxazine Maleate

A mixture of 4a,11b)-3,4,4a,5,6,11b-hexahydro-2*H*-[1]benzoxepino[5,4-*b*]-1,4-oxazine, prepared as in the preceeding Example, 3.0 ml of triethylamine and 25 ml of methylene chloride is stirred and a solution of 1.0 ml of ethyl chloroformate dissolved in 25 ml of methylene chloride is added dropwise thereto. The mixture is stirred overnight, washed with a dilute hydrochloric acid solution, followed by a saturated sodium chloride wash, and dried over magnesium sulfate. The solvent is evaporated and the residue is dissolved in 25 ml of tetrahydrofuran.

This solution is added dropwise to a suspension of 1.0 g of lithium aluminum hydride in 50 ml of tetrahydrofuran. The resulting mixture is refluxed for 6 hours, cooled in an ice bath, and excess hydride is decomposed via the cautious addition of 4.0 ml of a 10% aqueous solution of sodium hydroxide. The mixture is stirred overnight, filtered, and the filtrate evaporated to dryness. The residue is dissolved in ether, treated with an ethereal solution of maleic acid to form the maleate salt. Recrystallization of this salt from a methanol/ethyl acetate mixture yields the compound, *trans*-(4a,11b)-3,4,4a,5,6,11b-hexahydro-4-methyl-2*H*-[1]benzoxepino[5,4-*b*]-1,4-oxazine maleate having a m.pt. of 153—4°C.

Following essentially the same procedure but substituting *trans*-(4a,11b)-10-chloro-3,4,4a,5,6-11b-hexahydro-2*H*-[1]benzoxepino[5,4-*b*]-1,4-oxazine for the *trans*-(4a,11b)-3,4,4a,5,6,11b-hexahydro-2*H*-[1]-benzoxepino-[5,4-*b*]-1,4-oxazine above, results in the formation of *trans*-(4a,11b)-3,4,4a,5,6,11b-hexahydro-4-methyl-2*H*-[1]benzoxepino[5,4-*b*]-1,4-oxazine maleate having a m.pt. of 142—3°C.

## Example 3

Trans-(4a,11b)-3,4,4a,5,6,11b-Hexahydro-N,N-dimethyl-2H-[1]benzoxepino[5,4-b]-1,4-oxazine-4-ethanamine Maleate

A solution of 1.21 g of *trans*-(4a,11b)-3,4,4a,5,6,11b-hexahydro-2*H*-[1]benzoxepino[5,4-*b*]-1,4-oxazine hydrochloride, prepared according to the procedure of Example 1, and 1.65 ml of triethylamine in 25 ml of methylene chloride is treated dropwise with a solution of 0.70 ml of chloroacetyl chloride dissolved in 10 ml of methylene chloride. The reaction mixture is stirred overnight at room temperature, washed with a dilute solution of hydrochloric acid, followed by a water wash, a wash of dilute sodium hydroxide solution and finally by a wash of a saturated solution of sodium chloride. The reaction mixture is dried over anhydrous magnesium sulfate and evaporated to dryness to obtain 1.44 g of *trans*-(4a,11b)-3,4,4a,5,6,11b-hexahydro-2*H*-[1]benzoxepino[5,4-*b*]-1,4-oxazine.

The *N*-chloroacetyl derivative above is dissolved in 50 ml of ethanol containing 1.5 grams of dimethylamine and the solution refluxed for approximately 4 hours. The reaction mixture is evaporated to dryness and the residue is taken up with ether. The ether solution is washed with water, followed by a saturated, aqueous brine solution and is dried over anhydrous magnesium sulfate. Treatment of the dried solution with ethereal hydrogen chloride results in the preparation of the *N*-dimethylaminoacetyl derivative as the hydrochloride salt.

To a stirred suspension of 1.0 g of lithium aluminum hydride in 50 ml of tetrahydrofuran is added the above hydrochloride salt of the *N*-dimethylaminoacetyl derivative in small portions. The mixture is refluxed for 2 hours under an inert atmosphere of argon, cooled in an ice bath, and cautiously decomposed by the addition of 1.5 ml of water. The mixture is stirred overnight, filtered and the filtrate is evaporated to dryness. The residue is dissolved in ether and treated with a solution of maleic acid in ether to form the maleate salt. Recrystallization of this salt from a methanol/ethyl acetate mixture yields the compound, *trans* - (4a,11b) - 3,4,4a,5,6,11b - hexahydro - *N,N* - dimethyl - 2*H* - [1]benzoxepino[5,4-*b*] - 1,4 - oaxine - 4 - ethanamine as the maleate salt, having a m.pt. of 116—121°C.

## Example 4

Trans-(4a,11b)-3,4,4a,5,6,11b-hexahydro-2H-[1]benzoxepino[5,4-b]-1,4-oxazine

To a stirred solution of 2.41 g of *trans*-(4a,11b)-3,4,4a,5,6,11b-hexahydro-2*H*-[1]benzoxepino[5,4-*b*]-1,4-oxazine, prepared in accordance with Example 1, and 3.5 ml of triethylamine dissolved in 50 ml of methylene chloride is added dropwise a solution of 0.85 ml of acetyl chloride dissolved in 25 ml of methylene chloride. The mixture is stirred overnight, washed with a dilute hydrochloric acid solution and dried over anhydrous magnesium sulfate. The volatiles are removed by evaporation leaving an oil which yields the compound *trans*-(4a,11b)-3,4,4a,5,6,11b-hexahydro-2*H*-[1]benzoxepino[5,4-*b*]-1,4-oxazine having a m.pt. of 97—8°C .

To 1.38 g of this 4-acetyl derivative dissolved in 15 ml of tetrahydrofuran is added dropwise a suspension of 0.44 g of lithium aluminum hydride in 25 ml of tetrahydrofuran. The reaction mixture is refluxed for a period of about 5 hours, cooled in ice, cautiously decomposed using 1.2 ml of a 10% aqueous sodium hydroxide solution, and stirred overnight. The reaction mixture is filtered and the solvent evaporated *in vacuo* to an oil which upon vacuum distillation yielded 1.13 g of *trans*-(4a,11b)-3,4,4a,5,6,11b-hexahydro-2*H*-[1]benzoxepino[5,4-*b*]-1,4-oxazine as an oil having a b.pt. of 110—15°C 13,3 Pa (0.1 mm. of Hg).

Following essentially the same procedure but substituting the following acyl chlorides for the acetyl chloride above, results in the following products and/or their salts as shown below.

| Reactant | Product | m. pt. |
|---|---|---|
| benzoyl chloride | (4a,11b)-3,4, 4a,5,6,11b-hexahydro-4-(phenylmethyl)-2*H*-[1]-benzoxepino[5,4-*b*]-1,4-oxazine hydrochloride | |
| propionyl chloride | (4a,11b)-3,4,4a, 5,6,11b-hexahydro-4-propyl-2*H*-[1]-benzoxepino[5,4-*b*]-1,4-oxazine 4-methylbenzenesulfonate | 181—5°C |
| isobutyryl chloride | (4a,11b)-3,4,4a, 5,6,11b-hexahydro-4-(2-methylpropyl)-2*H*-[1]benzoxepino-[5,4-*b*]-1,4-oxazine 4-methylbenzenesulfonate | 190—3-C |
| 2-furoyl chloride | (4a,11b)-4-(2-furanylmethyl)-3,4,4a,5,6,11b-hexahydro-2*H*-[1]-benzoxepino[5,4-*b*]-1,4-oxazine hydrochloride | 254—6°C (dec.) |
| methoxyacetyl chloride | (4a,11b)-3,4,4a, 5,6,11b-hexahydro-4-(2-methoxyethyl)-2*H*-[1]benzoxepino[5,4-*b*]-1,4-oxazine | 110—15°C 80 Pa (0.6 mm) |
| cyclopropanecarboxylic acid chloride | (4a,11b)-4-cyclopropylmethyl-3,4,4a, 5,6,11b-hexahydro-2*H*-[1]benzoxepino[5,4-*b*]-1,4-oxazine hydrochloride | 254—6°C |

Example 5

Cis and trans-4-Amino-2,3,4,5-tetrahydrobenzoxepin-5-ol

A stirred suspension of 43.84 g (0.2 M) of 4-acetamido-2,3-dihydrobenzoxepin-5(4H)one in 60 ml of ethanol was cooled in ice and 10 g of sodium borohydride was added portionwise. After five hours the solvent was removed and the residue dissolved in 800 ml of water. Extraction with $CHCl_3$ gave a mixture of *cis* and *trans*-4-acetamido-2,3,4,5-tetrahydrobenzoxepin-5-ol as an oil.

A mixture of the above oil (10.76 g), 25 g of 50% sodium hydroxide and 50 ml of ethanol was refluxed for five hours, cooled and diluted to 400 ml with water. On standing overnight, *trans*-4-amino-2,3,4,5-tetrahydrobenzoxepin-5-ol precipitated, mp 144—146°. An analytical sample was recrystallized from toluene, mp. 146—148°.

Concentration of the filtrate gave crude *cis*-4-amino-2,3,4,5-tetrahydrobenzoxepin-5-ol. Several recrystallization from ethyl acetate gave the pure *cis*-amino alcohol, mp. 136—138°.

Example 6

4a,5,6,11b-Tetrahydro(4a,11b)-2H[1]benzoxepino[5,4b]-1,4-oxazin-3(4H)one

A solution of 0.1 M of *cis*-4-amino-2,3,4,5-tetrahydrobenzoxepin-5-ol and 0.11 M of triethylamine in 200 ml of methylene chloride was cooled in ice and 0.1 M of chloroacetyl chloride in 50 ml of methylene chloride was added dropwise. After stirring overnight at room temperature, the solution was extracted with dilute hydrochloric acid and the solvent evaporated. The residue was stirred with 250 ml of isopropanol

and sodium hydroxide (2 equivalents as a 50% aqueous solution) was added. The mixture was stirred overnight, concentrated and diluted with water to give the title product. An analytical sample recrystallized from ethyl acatate had mp. 156—164°.

Example 7

3,4,4a,5,6,11b-Hexahydro(4a,11b)2H[1]benzoxepino[5,4b]-1,4-oxazine maleate

4a,5,6,11b-tetrahydro(4a,11b)-2H[1]benzoxepino[5,4b]-1,4-oxazine-3(4H)one (0.01 M) was added via a solution funnel to a suspension of 0.005 M lithium aluminum hydride in 100 ml of dry tetrahydrofuran. The mixture was refluxed for five hours, cooled in ice and decomposed with 15% NaOH. After stirring overnight the mixture was filtered and the solvent removed from the filtrate. The residue was dissolved in ether and treated with a solution of maleic acid in ether to give the title compound. An analytical sample was recrystallized from ethyl acetate, mp. 197—198° (dec).

Example 8

4-Ethyl-3,4,4a,5,6,11b-Hexahydro(4a,11b)2H[1]benzoxepino[5,4b]-1,4-oxazine

A solution of 0.01 M of 3,4,4a,5,6,11b-hexahydro-(4a,11b)2H[1]benzoxepino[5,4b]-1,4-oxazine and 0.011 M of triethylamine in 25 ml of methylene chloride was cooled in ice and treated with 0.01 M of acetyl chloride in 10 ml of methylene chloride. After stirring at room temperature overnight, the solution was extracted with dilute hydrochloric acid and dried over magnesium sulfate. Evaporation of the solvent left an oil which was dissolved in 10 ml of dry tetrahydrofuran and this solution added dropwise to a suspension of 0.01 M of lithium aluminum hydride in 25 ml of tetrahydrofuran. After refluxing five hours, the mixture was cooled in ice and decomposed with 15% sodium hydroxide. After stirring overnight, the solids were filtered and the solvent removed from the filtrate. Kugelrohr distillation of the residue at 110—116° 26.6 Pa (0.2 mm) gave the title compound as an oil.

Similarly, by using the foregoing techniques, the *cis* or *cis-trans* of the mixtures may be obtained for any of the foregoing specifically mentioned compounds or any of the compounds embraced by formula I

Example 9

Preparation of a tablet formulation

One thousand tablets for oral use, each containing 125 mg of (4a,11b)-3,4,4a,5,6-11b-hexahydro-2*H*-[1]benzoxepino[5,4-*b*]-1,4-oxazine hydrochloride are prepared according to the following formulation:

| | Ingredients | Gm |
|---|---|---|
| (a) | (4a,11b)-3,4,4a,5,6,11b-hexahydro-2*H*-[1]benzoxepino-[5,4-*b*]-1,4-oxazine hydrochloride | 125 |
| (b) | Dicalcium phosphate | 150 |
| (c) | Methylcellulose, U.S.P. (15 cps) | 6.5 |
| (d) | Talc | 20 |
| (e) | Calcium sterate | 2.5 |

The (4a,11b)-3,4,4a,5,6,11b-hexahydro-2*H*-[1]benzoxepino[5,4-*b*]-1,4-oxazine hydrochloride and dicalcium phosphate are mixed well, granulated with a 7.5% aqueous solution of methylcellulose, passed through a No. 8 screen and carefully dried. The dried granules are passed through a No. 12 screen, blended with talc and calcium stearate and compressed into tablets.

Example 10

Preparation of a capsule formulation

One thousand two-piece hard gelatin capsules for oral use each containing 200 mg of (4a,11b)-3,4,4a,5,6,11b-hexahydro-4-methyl-2*H*-[1]benzoxepino[5,4-*b*]-1,4-oxazine maleate are prepared from the following ingredients:

| | Ingredients | Gm |
|---|---|---|
| (a) | (4a,11b)-3,4,4a,5,6,11b-hexahydro-4-methyl-2*H*-[1]-benzoxepino[5,4-*b*]-1,4-oxazine maleate | 200 |
| (b) | Lactose, U.S.P. | 100 |
| (c) | Starch U.S.P. | 10 |
| (d) | Talc, U.S.P. | 5 |
| (e) | Calcium sterate | 1 |

The finely powdered materials are mixed until uniformly dispersed and filled into hard shelled gelatin capsules of the appropriate size.

In a similar fashion one-piece soft gelatin capsules can be prepared in which the above formulation can be granulated, slugged or compressed directly into a rotary die or plate mold in which the capsule is formed. Alternatively, the above excipients may be omitted and the active ingredient dispensed as a powder directly into the capsule.

## Example 11

The following example illustrates the muscle relaxant activity obtained with the compound of this invention.

### Antagonism of Morphine-induced Straub Tail

The characteristic elevation of the mouse tail (Straub tail) following morphine administration is due to a sustained, centrally mediated reflux contraction of the sacrococcygeus dorsalis muscle. Five to ten mice are used in each test, weighed (18 to 30 grams) and placed in a plexiglass observation chamber 2 to 3 minutes prior to injection. Animals are treated with the test compound at log doses, ca. 10 ml/kg, based upon the $ED_{50}$ obtained from the Rotorod test (Reduction of motor ability). Fifteen minutes later the desired dose of morphine sulfate, ca. 10 ml/kg, is injected subcutaneously and the mice observed for a period of 30 minutes following morphine administration for the presence or absence of Straub tail (defined as elevation of tail at 90° or more).

### Reduction of Motor Ability

Following a method of Kinnard and Carr, J. Pharmacol. Exp. Therap. *121*, 354 (1957), groups of 10 mice are pre-selected for their ability to remain upon a horizontal rotating (15 rpm) rod (rotorod) for 120 seconds. The mice are dosed with the test compound and tested at various times. The percent change in mean time upon the rotored from the pre-drug control time of 120 seconds is calculated for each dose group. These data are analyzed by computer using a linear regression program to estimate the $ED_{50}$ with 95% confidence limits and slope. The $ED_{50}$ is defined as the dose of compound producing a 50% decrease in mean time on the rotorod.

Results:

| Compound* | Straub Tail $ED_{50}$ (mg/kg i.p.) | Rotorod $ED_{50}$ (mg/kg i.p.) |
|---|---|---|
| (4a,11b)-3,4,4a,5,6,11b-hexahydro-2*H*-[1]benzoxepino[5,4-*b*]-1,4-oxazine hydrochloride | 1.03 | 23.9 |
| (4a,11b)-3,4,4a,5,6,11b-hexahydro-4-methyl-2*H*-[1]benzoxepino-[5,4-*b*]-1,4-oxazine maleate | 2.89 | 12.4 |

* *Trans* isomers

Example 12

The following example illustrates the analgesic activity obtained with the compounds of this invention.

Groups of 5 to 10 mice are administered one or more doses of the test compound by the desired route (but not intraperitoneally). At a selected time the mice are administered acetic acid, 0.4 ml (0.25% v/v solution) i.p. Five minutes later, the mice are observed for a period of 15 minutes to determine the appearance of squirming (abdominal writhing), and the number of squirms for each mouse is determined.

Analgesia is considered significant in those mice which do not squirm during the 15 minute observation period. For $ED_{50}$ determination, four or more doses of compound are tested in groups of 10 mice. The following $ED_{50}$'s for analgesic activity were observed and determined.

| Compound* | Pretreatment Time (minutes) | $ED_{50}$ (mg/kg) Subcutaneous | oral |
|---|---|---|---|
| (4a,11b)-3,4,4a,5,6,11b-hexahydro-4-methyl-2*H*-[1]benzoxepino-[5,4-*b*]-1,4-oxazine maleate | 30 | 4.35 | 42.8 |
| | 60 | 13.8 | 52.2 |
| | 120 | 26.6 | [50% @ 256 mg/kg] |
| (4a,11b)-4-ethyl-3,4,4a,5,6,11b-hexahydro-2*H*-[1]-benzoxepino[5,4-*b*]-1,4-oxazine | 30 | 1.5 | 6.8 |
| | 60 | 8.8 | — |
| | 120 | >128 | — |

* *Trans* isomers

**Claims for the Contracting States: BE CH DE FR GB IT LI LU NL SE**

1. A (4a,11b)-3,4,4a,5,6,11b-hexahydro-2*H*-[1]benzoxepino[5,4-*b*]-1,4-oxazine having the general formula I

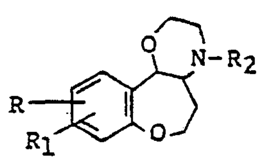

(I)

including their *cis* and *trans* isomers and enantiomeric forms thereof, wherein R and $R_1$ are each hydrogen, fluoro, chloro or $C_1$—$C_4$-alkyl;

$R_2$ is hydrogen, $C_1$—$C_4$-alkyl, phenyl-$C_1$—$C_4$-alkyl, $C_1$—$C_4$-alkoxy-$C_1$—$C_4$-alkyl, cyclopropylmethyl, 2-furanylmethyl or

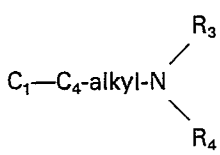

wherein $R_3$ and $R_4$ are each hydrogen or $C_1$—$C_4$-alkyl; and the pharmaceutical acceptable salts thereof.

2. A compound according to claim 1 wherein $R_2$ is hydrogen.

3. A compound according to claim 1 wherein $R_2$ is $C_1$—$C_4$-alkyl.

4. A compound according to claim 1 wherein R and $R_1$ are hydrogen.

5. A compound according to claim 1 which is *trans*-(4a,11b)-3,4,4a,5,6,11b-hexahydro-2*H*-[1]-benzoxepino[5,4-*b*]-1,4-oxazine and its pharmaceutically acceptable salts.

6. A compound according to claim 1 which is *trans*-(4a,11b)-3,4,4a,5,6,11b-hexahydro-4-methyl-2*H*-[1]-benzoxepino[5,4-*b*]-1,4-oxazine and its pharmaceutically acceptable salts.

7. A compound according to claim 1 which is *trans*-(4a,11b)-3,4,4a,5,6,11b-hexahydro-4-(phenylmethyl)-2*H*-[1]benzoxepino[5,4-*b*]-1,4-oxazine and its pharmaceutically acceptable salts.

8. A process for the preparation of a compound having the general formula I according to claim 1, which comprises:

(1) reacting a 4-amino-2,3,4,5-tetrahydro-1-benzoxepin-5-ol having the general formula II

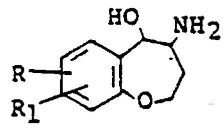

(II)

with chloroacetyl chloride in the presence of a trialkylamine to prepare a 2′-chloro-*N*-(2,3,4,5-tetrahydro-5-hydroxy-1-benzoxepin-4-yl)acetamide;
cyclizing said acetamide with sodium hydroxide to prepare a (4a,11b)-4a,5,6-11b-tetrahydro-2*H*-[1]benzoxepino[5,4-*b*]-1,4-oxazine-3(4H)one having the general formula IV

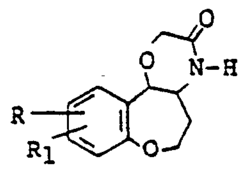

(IV)

and reducing said 1,4-oxazine-3(4H)one using a hydride reagent or diborane in an inert organic solvent to prepare a (4a,11b)-3,4,4a,5,6,11b-hexahydro-2*H*-[1]benzoxepino[5,4-*b*]-1,4-oxazine having the general formula V (a compound of the general formula I wherein $R_2$ is hydrogen)

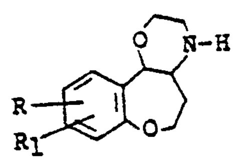

(V)

(2) for preparing a compound of the general formula I, wherein $R_2$ is other than hydrogen or methyl, acylating said benzoxepino[5,4-*b*]-1,4-oxazine of formula V with an acid chloride or an acid anhydride in the presence of a trialkylamine to prepare an *N*-acyl-(4a,11b)-3,4,4a,5,6,11b-hexahydro-2*H*-[1]benzoxepino[5,4-*b*]-1,4-oxazine having the general formula VI

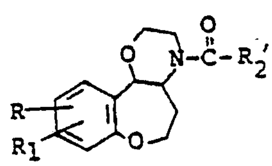

(VI)

wherein $R_2'$ is defined as $R_2$ above however containing one methylene group less than $R_2$;
and reducing said *N*-acyl-benzoxepino[5,4-*b*]-1,4-oxazine using a hydride reagent or diborane in an inert organic solvent and recovering the desired (4a,11b)-3,4,4a,5,6,11b-hexahydro-2*H*-[1]-benzoxepino[5,4-*b*]-1,4-oxazine therefrom, or
(3) for preparing a compound of the general formula I, wherein $R_2$ is methyl, reacting said benzoxepino[5,4-b]-1,4-oxazine of formula V with an alkyl chloroformate in the presence of a trialkylamine to prepare a (4a,11b)-3,4,4a,5,6,11b-hexahydro-4-carboalkoxy-2*H*-[1]-benzoxepino[5,4-*b*]-1,4-oxazine derivative of the general formula VIII

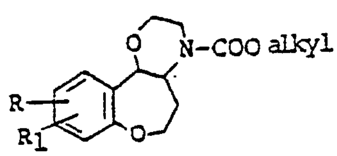

(VIII)

and reducing said benzoxepino[5,4-b]-1,4-oxazine derivative using a hydride reagent or diborane in an inert organic solvent and recovering the desired 4-methyl derivative.

9. Pharmaceutical composition comprising a compound as set forth in claim 1.

**Claims for the Contracting State: AT**

1. A process for the preparation of a (4a,11b)-3,4,4a,5,6,11b-hexahydro-2*H*-[1]benzoxepino[5,4-*b*]-1,4-oxazine having the general formula I

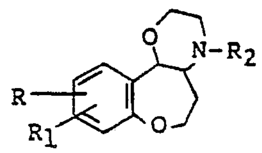

(I)

including their *cis* and *trans* isomers and enantiomeric forms thereof, wherein R and $R_1$ are each hydrogen, fluoro, chloro or $C_1$—$C_4$-alkyl, phenyl-$C_1$—$C_4$-alkyl;
$R_2$ is hydrogen, $C_1$—$C_4$-alkyl, $C_1$—$C_4$-alkoxy-$C_1$—$C_4$-alkyl, cyclopropylmethyl, 2-furanylmethyl or

$R_3$
/
$C_1$—$C_4$-alkyl-N
\
$R_4$

wherein $R_3$ and $R_4$ are each hydrogen or $C_1$—$C_4$-alkyl; and the pharmaceutical acceptable salts thereof, which comprises

(1) reacting a 4-amino-2,3,4,5-tetrahydro-1-benzoxepin-5-ol having the general formula II

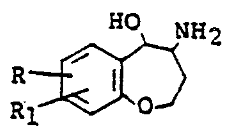

(II)

with chloroacetyl chloride in the presence of a trialkylamine to prepare a 2′-chloro-*N*-(2,3,4,5-tetrahydro-5-hydroxy-1-benzoxepin-4-yl)acetamide;
cyclizing said acetamide with sodium hydroxide to prepare a (4a,11b)-4a,5,6-11b-tetrahydro-2*H*-[1]benzoxepino[5,4-*b*]-1,4-oxazine-3(4H)one having the general formula IV

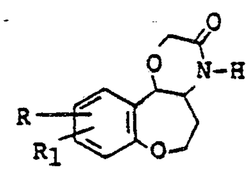

(IV)

and reducing said 1,4-oxazine-3(4H)one using a hydride reagent or diborane in an inert organic solvent to prepare a (4a,11b)-3,4,4a,5,6,11b-hexahydro-2*H*-[1]benzoxepino[5,4-*b*]-1,4-oxazine having the general formula V (a compound of the general formula I wherein $R_2$ is hydrogen)

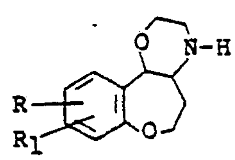

(V)

(2) for preparing a compound of the general formula I, wherein $R_2$ is other than hydrogen or methyl, acylating said benzoxepino[5,4-*b*]-1,4-oxazine of formula V with an acid chloride or an acid anhydride in the presence of a trialkylamine to prepare an *N*-acyl-(4a,11b)-3,4,4a,5,6,11b-hexahydro-2*H*-[1]benzoxepino[5,4-*b*]-1,4-oxazine having the general formula VI

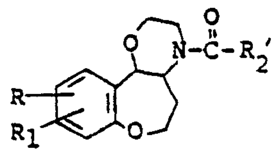

(VI)

wherein $R_2'$ is defined as $R_2$ above however containing one methylene group less than $R_2$; and reducing said *N*-acyl-benzoxepino[5,4-*b*]-1,4-oxazine using a hydride reagent or diborane in an inert organic solvent and recovering the desired (4a,11b)-3,4,4a,5,6,11b-hexahydro-2*H*-[1]-benzoxepino[5,4-*b*]-1,4-oxazine therefrom, or

(3) for preparing a compound of the general formula I, wherein $R_2$ is methyl, reacting said benzoxepino[5,4-b]-1,4-oxazine of formula V with an alkyl chloroformate in the presence of a trialkylamine to prepare a [4a,11b)-3,4,4a,5,6,11b-hexahydro-4-carboalkoxy-2*H*-[1]-benzoxepino[5,4-*b*]-1,4-oxazine derivative of the general formula VIII

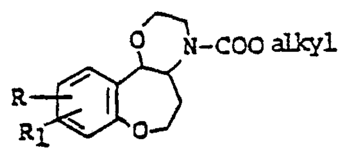

(VIII)

and reducing said benzoxepino[5,4-b]-1,4-oxazine derivative using a hydride reagent or diborane in an inert organic solvent and recovering the desired 4-methyl derivative.

2. The process according to claim 1 for the production of *trans*-(4a,11b)-3,4,4a,5,6,11b-hexahydro-2*H*-[1]benzoxepino[5,4-*b*]-1,4-oxazine and its pharmaceutically acceptable salts.

3. The process according to claim 1 for the production of *trans*-(4a,11b)-3,4,4a,5,6,11b-hexahydro-4-methyl-2*H*-[1]benzoxepino[5,4-*b*]-1,4-oxazine and its pharmaceutically acceptable salts.

4. The process according to claim 1 for the production of *trans*-(4a,11b)-3,4,4a,5,6,11b-hexahydro-4-(phenylmethyl)-2*H*-[1]benzoxepino[5,4-*b*]-1,4-oxazine and its pharmaceutically acceptable salts.

**Patentansprüche für die Vertragsstaaten: BE CH DE FR GB IT LI LU NL SE**

1. (4a,11b)-3,4,4a,5,6,11b-Hexahydro-2H-[1]benzoxepino[5,4-b]-1,4-Oxazin der allgemeinen Formel I

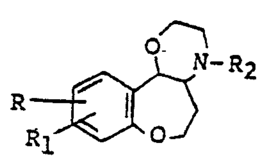

(I)

einschließlich seiner cis- und trans-Isomere und enantiomere Formen davon, in der R und $R_1$ jeweils ein Wasserstoff-, Fluor- oder Chloratom oder einen $C_1$—$C_4$-Alkylrest bedeuten;
$R_2$ ein Wasserstoffatom, einen $C_1$—$C_4$-Alkyl-, Phenyl-$C_1$—$C_4$-alkyl-, $C_1$—$C_4$-Alkoxy-$C_1$—$C_4$-alkyl-, Cyclopropylmethyl-, 2-Furanylmethyl- oder

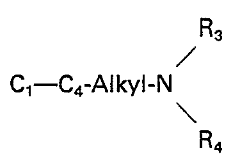

-Rest bedeutet, wobei $R_3$ und $R_4$ jeweils ein Wasserstoffatom oder einen $C_1$—$C_4$-Alkylrest darstellen, und pharmakologisch verträgliche Salze davon.

2. Verbindung nach Anspruch 1, in der $R_2$ ein Wasserstoffatom darstellt.

3. Verbindung nach Anspruch 1, in der $R_2$ einen $C_1$—$C_4$-Alkylrest darstellt.

4. Verbindung nach Anspruch 1, in der R und $R_1$ Wasserstoffatome sind.

5. Verbindung nach Anspruch 1, nämlich trans-(4a,11b)-3,4,4a,5,6,11b-Hexahydro-2H-[1]benzoxepino-[5,4-b]-1,4-oxazin und sein pharmakologisch verträglichen Salze.

6. Verbindung nach Anspruch 1, nämlich trans-(4a,11b)-3,4,4a,5,6,11b-Hexahydro-2H-[1]benzoxepino-[5,4-b]-1,4-oxazin und sein pharmakologisch verträglichen Salze.

7. Verbindung nach Anspruch 1, nämlich trans-(4a,11b)-3,4,4a,5,6,11b-Hexahydro-2H-[1]benzoxepino-[5,4-b]-1,4-oxazin und sein pharmakologisch verträglichen Salze.

8. Verfahren zur Herstellung einer Verbindung der allgemeinen Formel I nach Anspruch 1, dadurch gekennzeichnet, daß man
(1) ein 4-Amino-2,3,4,5-tetrahydro-1-benzoxepin-5-ol der allgemeinen Formel II

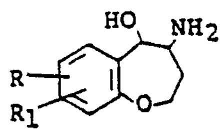

(II)

mit Chloracetylchlorid in Gegenwart eines Trialkylamins zur Herstellung eines 2'-Chlor-N-(2,3,4,5-tetrahydro-5-hydroxy-1-benzoxepin-4-yl)-acetamids umsetzt;
das Acetamid mit Natriumhydroxid zu einem (4a,11b)-4a,5,6,11b-Tetrahydro-2H-[1]benzoxepino[5,4-b]-1,4-oxazin-3(4H)-on der allgemeinen Formel IV

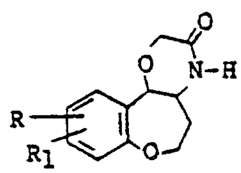

(IV)

cyclisiert und das 1,4-Oxazin-3(4H)-on mit einem Hydridreagens oder Diboran in einem inerten organischen Lösungsmittel zu einem (4a,11b)-3,4,4a,5,6,11b-Hexahydro-2H[1]benzoxepino[5,4-b]-1,4-oxazin der allgemeinen Formel V (eine Verbindung der allgemeinen Formel I, in der $R_2$ ein Wasserstoffatom ist)

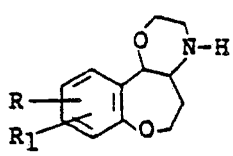

(V)

reduziert,
(2) zur Herstellung einer Verbindung der allgemeinen Formel I, in der $R_2$ weder ein Wasserstoffatom noch eine Methylgruppe ist, das Benzoxepino[5,4-b]-1,4-oxazin der allgemeinen Formel V mit einem Säurechlorid oder einem Säureanhydrid in Gegenwart eines Trialkylamins zu einem N-Acyl-(4a,11b)-3,4,4a,5,6,11b-hexahydro-2H-[1]benzoxepino[5,4-b]-1,4-oxazin der allgemeinen Formel VI

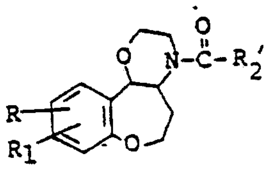

(VI)

in der $R_2'$ die vorstehend angegebene Bedeutung von $R_2$ hat, jedoch eine Methylengruppe weniger als $R_2$ enthält, acyliert;
und das N-Acyl-benzoxepino[5,4-b]-1,4-oxazin mit einem Hydridreagens oder Diboran in einem inerten organischen Lösungsmittel reduziert und das gewünschte (4a,11b)-3,4,4a,5,6,11b-Hexahydro-2H-[1]-benzoxepino[5,4-b]-1,4-oxazin daraus gewinnt, oder
(3) zur Herstellung einer Verbindung der allgemeinen Formel I, in der $R_2$ eine Methylgruppe darstellt, das Benzoxepino[5,4-b]-1,4-oxazin der allgemeinen Formel V mit einem Alkylchloroformat in Gegenwart von Trialkylamin zu einem (4a,11b)-3,4,4a,5,6,11b-Hexahydro-4-carboalkoxy-2H-[1]benzoxepino[5,4-b]-1,4-oxazin-Derivat der allgemeinen Formel VIII

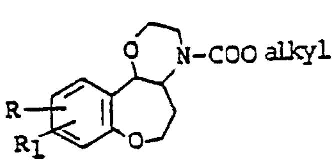

(VIII)

umsetzt und das Benzoxepino[5,4-b]-1,4-oxazin-Derivat mit einem Hydridreagens oder Diboran in einem inerten organischen Lösungsmittel reduziert und das gewünschte 4-Methyl-Derivat gewinnt.

9. Arzneimittel, gekennzeichnet durch den Gehalt an einer Verbindung nach Anspruch 1.

**Patentansprüche für den Vertragsstaat: AT**

1. Verfahren zur Herstellung eines (4a,11b)-3,4,4a,5,6,11b-Hexahydro-2H-[1]benzoxepino[5,4-b]-1,4-oxazins der allgemeinen Formel I

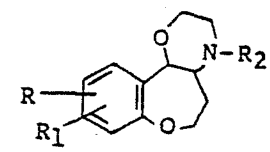

(I)

einschließlich seiner cis- und trans-Isomere und enantiomere Formen davon, in der R und $R_1$ jeweils ein Wasserstoff-, Fluor- oder Chloratom oder einen $C_1$—$C_4$-Alkylrest bedeuten;
$R_2$ ein Wasserstoffatom, einen $C_1$—$C_4$-Alkyl-, Phenyl-$C_1$—$C_4$-alkyl-, $C_1$—$C_4$-Alkoxy-$C_1$—$C_4$-alkyl-, Cyclopropylmethyl-, 2-Furanylmethyl- oder

$$C_1—C_4\text{-Alkyl-N}\begin{matrix} R_3 \\ \\ R_4 \end{matrix}$$

-Rest darstellt, wobei $R_3$ und $R_4$ jeweils ein Wasserstoffatom oder einen $C_1$—$C_4$-Alkylrest bedeuten, und pharmakologisch verträglicher Salze davon, dadurch gekennzeichnet, daß man
(1) ein 4-Amino-2,3,4,5-tetrahydro-1-benzoxepin-5-ol der allgemeinen Formel II

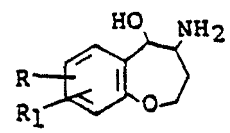

(II)

mit Chloracetylchlorid in Gegenwart eines Trialkylamins zu einem 2'-Chlor-N-(2,3,4,5-tetrahydro-5-hydroxy-1-benzoxepin-4-yl)-acetamid umsetzt;
das Acetamid mit Natriumhydroxid zu einem (4a,11b)-4a,5,6,11b-Tetrahydro-2H-[1]benzoxepino[5,4-b]-1,4-oxazin-3(4H)-on der allgemeinen Formel IV

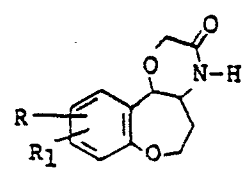

(IV)

cyclisiert,
und das 1,4-Oxazin-3(4H)-on mit einem Hydridreagens oder Diboran in einem inerten organischen Lösungsmittel zu einem (4a,11b)-3,4,4a,5,6,11b-Hexahydro-2H[1]benzoxepino[5,4-b]-1,4-oxazin der allgemeinen Formel V (eine Verbindung der allgemeinen Formel I, in der $R_2$ ein Wasserstoffatom ist)

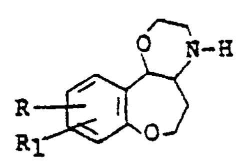

(V)

reduziert,
(2) zur Herstellung einer Verbindung der allgemeinen Formel I, in der $R_2$ weder ein Wasserstoffatom noch

eine Methylgruppe ist, das Benzoxepino[5,4-b]-1,4-oxazin der allgemeinen Formel V mit einem Säurechlorid oder einem Säureanhydrid in Gegenwart eines Trialkylamins zu einem N-Acyl-(4a,11b)-3,4,4a,5,6,11b-hexahydro-2H-[1]benzoxepino[5,4-b]-1,4-oxazin der allgemeinen Formel VI

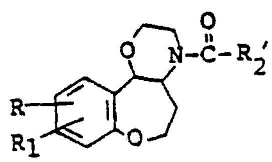

(VI)

in der $R_2'$ die vorstehend für $R_2$ angegebene Bedeutung hat, jedoch eine Methylengruppe weniger als $R_2$ enthält, acyliert;
und das N-Acyl-benzoxepino[5,4-b]-1,4-oxazin mit einem Hydridreagens oder Diboran in einem inerten organischen Lösungsmittel reduziert und das gewünschte (4a, 11b)-3,4,4a,5,6,11b-Hexahydro-2H-[1]-benzoxepino[5,4-b]-1,4-oxazin daraus gewinnt, oder
(3) zur Herstellung einer Verbindung der allgemeinen Formel I, in der $R_2$ eine Methylgruppe darstellt, das Benzoxepino[5,4-b]-1,4-oxazin der allgemeinen Formel V mit einem Alkylchloroformat in Gegenwart eines Trialkylamins zu einem (4a,11b)-3,4,4a,5,6,11b-Hexahydro-4-carboalkoxy-2H-[1]benzoxepino[5,4-b]-1,4-oxazin-Derivat der allgemeinen Formel VIII

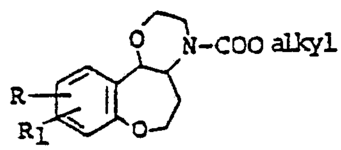

(VIII)

umsetzt und das Benzoxepino[5,4-b]1,4-oxazin-Derivat mit einem Hydridreagens oder Diboran in einem inerten organischen Lösungsmittel reduziert und das gewünschte 4-Methyl-Derivat gewinnt.

2. Verfahren nach Anspruch 1 für die Herstellung von trans-(4a,11b)-3,4,4a,5,6,11b-Hexahydro-2H-[1]benzoxepino-[5,4-b]-1,4-oxazin und seiner pharmakologisch verträglichen Salze.

3. Verfahren nach Anspruch 1, für die Herstellung von trans-(4a,11b)-3,4,4a,5,6,11b-Hexahydro-4-methyl-2H-[1]benzoxepino[5,4-b]-1,4-oxazin und seiner pharmakologisch verträglichen Salze.

4. Verfahren nach Anspruch 1 für die Herstellung von trans-(4a,11b)-3,4,4a,5,6,11b-Hexahydro-4(phenylmethyl)-2H-[1]benzoxepino[5,4-b]-1,4-oxazin und seiner pharmakologisch verträglichen Salze.

**Revendications pour les Etats contractants: BE CH DE FR GB IT LI LU NL SE**

1. Une (4a,11b)-3,4,4a,5,6,11b-hexahydro-2H-[1]benzoxépino[5,4-b]-1,4-oxazine répondant à la formule générale I:

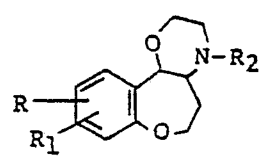

(I)

y compris les isomères cis et trans et leurs formes énantiomères, où R et $R_1$ sont chacun un hydrogène, un fluoro, un chloro ou un alkyle en $C_1$—$C_4$; $R_2$ est un hydrogène, un alkyle en $C_1$—$C_4$, un phényl-alkyle en $C_1$—$C_4$, un alcoxy($C_1$—$C_4$)alkyle en $C_1$—$C_4$, un cyclopropylméthyle, un 2-furanylméthyle ou un

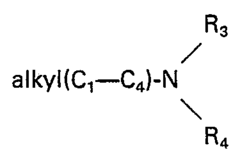

où $R_3$ et $R_4$ sont chacun un hydrogène ou un alkyle en $C_1$—$C_4$; et leurs sels convenant en pharmacie.

2. Un composé selon la revendication 1 où $R_2$ est un hydrogène.

3. Un composé selon la revendication 1 où $R_2$ est un alkyle en $C_1$—$C_4$.

4. Un composé selon la revendication 1 où R et $R_1$ sont un hydrogène.

5. Un composé selon la revendication 1 qui est la trans-(4a,11b)-3,4,4a,5,6,11b-hexahydro-2H-[1]benzoxépino[5,4-b]-1,4-oxazine et ses sels convenant en pharmacie.

6. Un composé selon la revendication 1 qui est la trans-(4a,11b)-3,4,4a,5,6,11b-hexahydro-4-méthyl-2H-[1]benzoxépino[5,4-b]-1,4-oxazine et ses sels acceptables en pharmacie.

7. Un composé selon la revendication 1 qui est la trans-(4a,11b)-3,4,4a,5,6,11b-hexahydro-4-(phénylméthyl)-2H-[1]benzoxépino[5,4-b]-1,4-oxazine et ses sels acceptables en pharmacie.

8. Un procédé pour la préparation d'un composé répondant à la formule générale I selon la revendication 1 qui comprend

(1) la réaction d'un 4-amino-2,3,4,5-tétrahydro-1-benzoxépine-5-ol répondant à la formule générale II

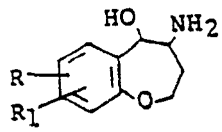

(II)

avec le chlorure de chloroacétyle en présence d'une trialkylamine pour préparer un 2'-chloro-N-(2,3,4,5-tétrahydro-5-hydroxy-1-benzoxépine-4-yl)acétamide;

la cyclisation dudit acétamide avec l'hydroxyde de sodium pour préparer une (4a,11b)-4a,5,6,11b-tétrahydro-2H-[1]benzoxépino[5,4-b]-1,4-oxazine-3(4H)one répondant à la formule générale IV:

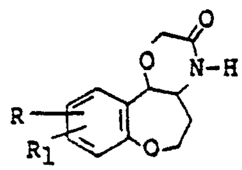

(IV)

et la réduction de ladite 1,4-oxazine-3(4H)one en utilisant un agent de type hydrure ou le diborane dans un solvant organique inerte pour préparer une (4a,11b)-3,4,4a,5,6,11b-hexahydro-2H-[1]benzoxépino[5,4-b]-1,4-oxazine répondant à la formule générale V (un composé de formule générale I où $R_2$ est un hydrogène)

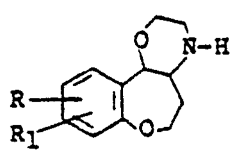

(V)

(2) pour préparer un composé de formule générale I où $R_2$ est autre qu'un hydrogène ou un méthyle, l'acylation de ladite benzoxépino[5,4-b]-1,4-oxazine de formule V avec un chlorure d'acide ou un anhydride d'acide en présence d'une trialkylamine pour préparer une N-acyl-(4a,11b)-3,4,4a,5,6,11b-hexahydro-2H-[1]benzoxépino[5,4-b]-1,4-oxazine répondant à la formule générale VI:

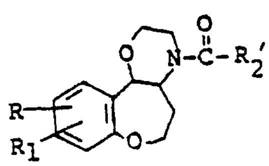

(VI)

où $R_2'$ est comme défini pour $R_2$ ci-dessus mais qui contient cependant un groupe méthylène de moins que $R_2$;

et la réduction de ladite N-acyl-benzoxépino[5,4-b]-1,4-oxazine avec un agent de type hydrure ou le diborane dans un solvant organique inerte et la récupération à partir de celui-ci de la (4a,11b)-3,4,4a,5,6,11b-hexahydro-2H-[1]benzoxépino[5,4-b]-1,4-oxazine, ou

(3) pour préparer un composé de formule générale I, où $R_2$ est un méthyle, la réaction de ladite benzoxépino[5,4-b]-1,4-oxazine de formule V avec un chloroformiate d'alkyle en présence d'une triéthylamine pour préparer un dérivé de (4a,11b)-3,4,4a,5,6,11b-hexahydro-4-carbalcoxy-2H-[1]benzoxépino[5,4-b]-1,4-oxazine de formule générale VIII:

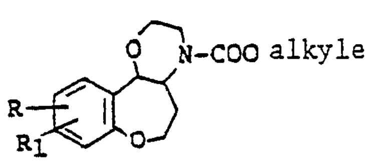

(VIII)

*et la réduction dudit dérivé de benzoxépino[5,4-b]-1,4-oxazine par emploi d'un agent de type hydrure ou du* diborane dans un solvant organique inerte et la récupération du dérivé 4-méthylé désiré.

9. Composition pharmaceutique comprenant un composé comme indiqué dans la revendication 1.

**Revendications pour l'Etat contractant: AT**

1. Un procédé pour la préparation d'une (4a,11b)-3,4,4a,5,6,11b-hexahydro-2H-[1]benzoxépino[5,4-b]-1,4-oxazine répondant à la formule générale I:

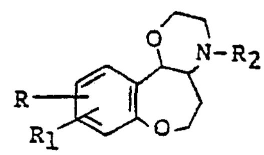

(I)

y compris les isomères cis et trans et leurs formes énantiomères, où R et $R_1$ sont chacun un hydrogène, un fluoro, un chloro ou un alkyle en $C_1$—$C_4$; $R_2$ est un hydrogène, un alkyle en $C_1$—$C_4$, un phényl-alkyle en $C_1$—$C_4$, un alcoxy($C_1$—$C_4$)alkyle en $C_1$—$C_4$, un cyclopropylméthyle, un 2-furanylméthyle ou

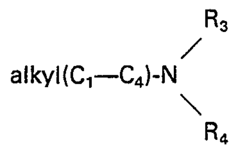

où $R_3$ et $R_4$ sont chacun un hydrogène ou un alkyle en $C_1$—$C_4$; et leurs sels convenant en pharmacie, qui comprend:

(1) la réaction d'un 4-amino-2,3,4,5-tétrahydro-1-benzoxépine-5-ol répondant à la formule générale II

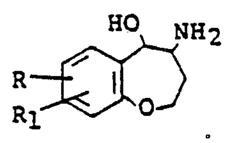

(II)

avec le chlorure de chloroacétyle en présence d'une trialkylamine pour préparer un 2'-chloro-N-(2,3,4,5-tétrahydro-5-hydroxy-1-benzoxépine-4-yl)acétamide;

la cyclisation dudit acétamide avec l'hydroxyde de sodium pour préparer une (4a,11b)-4a,5,6,11b-tétrahydro-2H-[1]benzoxépino[5,4-b]-1,4-oxazine-3(4H)one répondant à la formule générale IV:

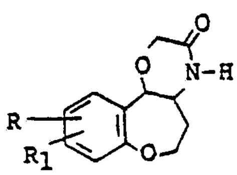

(IV)

et la réduction de ladite 1,4-oxazine-3(4H)one en utilisant un agent de type hydrure ou le diborane dans un solvant organique inerte pour préparer une (4a,11b)-3,4,4a,5,6,11b-hexahydro-2H-[1]benzoxépino[5,4-b]-1,4-oxazine répondant à la formule générale V (un composé de formule générale I où $R_2$ est un hydrogène):

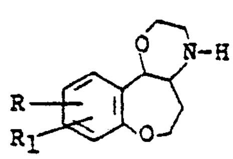

(V)

(2) pour préparer un composé de formule générale I où $R_2$ est autre qu'un hydrogène ou un méthyle, l'acylation de ladite benzoxépino[5,4-b]-1,4-oxazine de formule V avec un chlorure d'acide ou un anhydride d'acide en présence d'un trialkylamine pour préparer une N-acyl-(4a,11b)-3,4,4a,5,6,11b-hexahydro-2H-[1]-benzoxépino[5,4-b]-1,4-oxazine répondant à la formule générale VI:

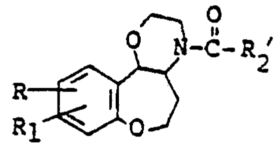

(VI)

où $R_2'$ est comme défini pour $R_2$ ci-dessus mais qui contient cependant un groupe méthylène de moins que $R_2$;

et la réduction de ladite N-acyl-benzoxépino[5,4-b]-1,4-oxazine avec un agent de type hydrure ou le diborane dans un solvant organique inerte et la récupération à partir de celui-ci de la (4a,11b)-3,4,4a,5,6,11b-hexahydro-2H-[1]benzoxépino[5,4-b]-1,4-oxazine, ou

(3) pour préparer un composé de formule générale I, où $R_2$ est un méthyle, la réaction de ladite benzoxépino[5,4-b]-1,4-oxazine de formule V avec un chloroformiate d'alkyle en présence d'une trialkylamine pour préparer un dérivé de (4a,11b)-3,4,4a,5,6,11b-hexahydro-4-carbalcoxy-2H-[1]benzoxépino[5,4-b]-1,4-oxazine de formule générale VIII:

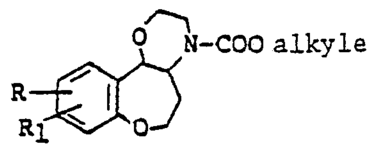

(VIII)

et la réduction dudit dérivé de benzoxépino[5,4-b]-1,4-oxazine par emploi d'un agent de type hydrure ou du diborane dans un solvant organique inerte et la récupération du dérivé 4-méthylé désiré.

2. Le procédé selon la revendication 1 pour la production de la trans-(4a,11b)-3,4,4a,5,6,11b-hexahydro-2H-[1]benzoxépino[5,4-b]-1,4-oxazine et de ses sels convenant en pharmacie.

3. Le procédé selon la revendication 1 pour la production de la trans-(4a,11b)-3,4,4a,5,6,11b-hexahydro-4-méthyl-2H-[1]benzoxépino[5,4-b]-1,4-oxazine et de ses sels convenant en pharmacie.

4. Le procédé selon la revendication 1 pour la production de la trans-(4a,11b)-3,4,4a,5,6,11b-hexahydro-4-(phénylméthyl)-2H-[1]benzoxépino[5,4-b]-1,4-oxazine et de ses sels convenant en pharmacie.